# EUROPEAN PATENT SPECIFICATION

(11) **EP 0 731 703 B1**
(45) Date of publication and mention of the grant of the patent: **10.09.2003**
(21) Application number: 95903468.7
(22) Date of filing: 06.12.1994
(51) Int. Cl.: A61K 31/70, A61K 9/127, A61P 31/12

(54) **LIPOSOMAL FORMULATIONS CONTAINING VIDARABINE OR ITS DERIVATIVES**
LIPOSOMFORMULIERUNGEN ENTHALTEND VIDARABIN ODER SEINE DERIVATE
FORMULATIONS LIPOSOMALES RENFERMANT DE LA VIDARABINE OU SES DERIVES

(30) Priority: 06.12.1993 PT 10142293
(43) Date of publication of application: 18.09.1996
(73) Proprietor: INSTITUTO NACIONAL DE ENGENHARIA E TECNOLOGIA INDUSTRIAL/INSTITUTO DE BIOTECNOLOGIA, QUIMICA FINA E TECNOLOGIAS ALIMENTARES, 1699 Lisboa Codex (PT); Meirinhos da Cruz, Maria Eugénia, 1500 Lisboa (PT); Goncalves Barbosa, Carlos Mauricio, 4100 Porto (PT); Cabral Caldeira, Luis Filipa Diniz, 1500 Lisboa (PT)
(72) Inventor: MEIRINHOS DA CRUZ, Maria, Eugénia, P-1500 Lisboa (PT); GON ALVES BARBOSA, Carlos, Mauricio, S. Jo o da Foz P-4100 Porto (PT); CABRAL CALDEIRA, Luis, Filipe, Diniz, P-1500 Lisboa (PT)
(74) Representative: Alves Moreira, Pedro
(86) International application number: PT9400013
(87) International publication number: WO95015762

(56) References cited:
- WO-A-88/00824
- WO-A-92/11037
- WO-A-94/20072
- CHEMICAL ABSTRACTS, vol. 119, no. 20, 15 November 1993, Columbus, Ohio, US; abstract no. 210899, & ZHONGGUO YIYAO GONGYE ZAZHI, vol.24, no.4, 1993 pages 160 - 164 Y. XUE ET AL. 'QUANTIFICATION AND ENCAPSULATION PERCENTAGE OF VIDARABINE IN LIPOSOME'
- CHEMICAL ABSTRACTS, vol. 116, no. 20, 18 May 1992, Columbus, Ohio, US; abstract no. 201019, & YAOXUE XUEBAO, vol.27, no.1, 1992 pages 74 - 80 Y. Y. XUE ET AL. 'THE PREPARATION AND STABILITY OF LIPOSOME -ENCAPSULATED ARA-A'
- CHEMICAL ABSTRACTS, vol. 101, no. 24, 10 December 1984, Columbus, Ohio, US; abstract no. 216332, & LIPOSOME TECHNOL., vol.2, 1984 pages 19 - 31 E. MAYHEW ET AL. 'PREPARATION OF LIPOSOMES ENTRAPPING CANCER CHEMOTHERAPEUTIC AGENTS FOR EXPERIMENTAL IN VIVO AND IN VITRO STUDIES'

## Description

### Background of the invention

This invention relates to the preparation of liposomal formulations containing vidarabine and/or a vidarabine derivative, such as vidarabine monophosphate (ARA-AMP), and mixtures of glycerophospholipids of saturated and unsaturated acyl chains, with cholesterol (CHOL) and charged molecules, lipidic or not (e.g. phosphatidylinositol (PI), phosphatidylglycerol (PG), stearylamine (SA), phosphatidic acid (PA)).

Liposomes are biocompatible and biodegradable microvesicular systems (Gregoriadis and Florence 1993), consisting on one or several phospholipid bilayers surrounding discrete aqueous compartments. These vesicles are able to incorporate all kinds of substances, such as drugs. Depending on its polarity, a compound is entrapped in a liposome either within the internal aqueous compartments or in the hydrophobic regions of the lipid bilayer. Liposomes can be prepared as sterile or pyrogen-free suspensions in submicron diameters to enable them to be injected intravenously. Because of their structural versatility in terms of size, lipid composition, surface charge. bilayer fluidity and ability to incorporate any drug regardless of solubility, or to carry ligands on their surface, liposomes have the potential to be tailored, in a variety of ways, to modulate their pharmacokinetic characteristics and to ensure the production of formulations that are optimal for clinical use.

Vidarabine (9-β-D-arabinofuranosyladenine, adenine arabinoside, ARA--A) and its more water-soluble derivative vidarabine monophosphate (9-β-D--arabinofuranosyladenine monophosphate, ARA-AMP) are purine nucleosides that inhibit DNA polymerase in viruses and to a lesser extent in mammals. These agents have been shown to have *in vitro* antiviral activity against a large number of DNA viruses. such as *Herpes simplex* types I and II, *Varicella-zoster*, *Epstein-Barr, Cytomegalovirus. Vaccinia* and Hepatitis B virus (HBV) (Shannon 1975). The clinical usefulness of ARA-A is limited by its poor aqueous solubility (Schabel 1968), which results in having to be administered by intravenous infusion in large volumes of fluid. On the contrary, ARA-AMP is water-soluble (LePage et al. 1972) and can be administered intravenously or intramuscularly in a small volume of injection fluid.

Persistent HBV infections are widely spread over the world, but unevenly distributed, with prevalence rates from 10-20% in coastal regions of China, in Southeast Asia and sub-Saharan Africa, to less than 0.5% in Northwest Europe, North America and Australia. It has been recently estimated that worldwide there are nearly 300 million actively infective carriers of HBV markers (Ayoola et al. 1988; Gerin 1992). This infection is the main cause of chronic liver disease and constitute a major cause of morbidity and mortality all around the world. Thus, although the infection can be prevented by vaccination, there is a need to develop effective forms of antiviral therapy for existing carriers. to reduce the squelae, which include chronic active hepatitis, cirrhosis and hepatocellular carcinoma (Tiollais et al. 1990; Lunel-Fabiani 1991; Marcellin 1991).

ARA-A and ARA-AMP are two of the few drugs with proven efficiency in the treatment of human chronic type B hepatitis. Several investigators have used either ARA-A or ARA-AMP by themselves (Weller 1986; Trepo et al. 1986; Guardia et al. 1986; Thomas 1988: Marcellin et al. 1989) or in combination with other therapeutic modalities (Scullard et al. 1981; Smith et al. 1982; Yokosuka et al. 1985; Guillevin et al. 1993) to treat patients with chronic hepatitis due to HBV. Both of these drugs displayed a constant effect in blocking virus growth, promoting a consistent decrease of the replication viral markers during treatment and reducing, in a dose dependent fashion, the serum levels of virus DNA-polymerase. Although ARA-A and ARA-AMP have constantly been shown to display an improvement in liver function and histology and a loss of infectivity in a significant proportion of patients, their use have produced conflicting results in terms of seroconversion rates and long-term efficacy. In fact, the therapeutic application of vidarabine is severely restricted by its toxicity. The cumulative dose-dependent side effects, which have been observed with therapeutic levels of ARA-A and ARA-AMP. include mainly neurological disturbances. such as the neuromuscular pain syndrome. and haematological and gastrointestinal disturbances (Sacks et al. 1982: Whitiey et al. 1980-a and 1980-b; Kanterewicz et al. 1990; Lok 1990). These problems constitute a major drawback, necessitating discontinuation of treatment and hindering the progress of clinical studies.

With the aim of increasing the chemotherapeutic index of vidarabine, Fiume et al. (1980) coupled either ARA-A or ARA-AMP to asialofetuin, a galactosyl terminating neoglycoprotein that selectively penetrates into hepatocytes by receptor-mediated endocytosis. A major drawback in the clinical use of these conjugates was their immunogenicity, which was overcome by using lactosaminated homologous albumin as hepatotropic carrier of ARA-AMP (Fiume et al. 1981, 1982-a, 1985, 1986-a, 1987, 1988-a, 1988-b). After injection of this conjugate in infected mice (Flume et al. 1981, 1982-b, 1986-a) and woodchucks (Ponzetto et al. 1991), synthesis of viral DNA was inhibited at lower concentrations than the uncoupled drug. In a preliminary clinical study, the conjugate administered for 3 days to patients with chronic HBV infection, inhibited virus replication (Flume et al. 1988-c). This strategy may reduce the dose-related side effects. However, the carrier is not generally available and, to be injected in humans, must be obtained from the human plasma, what presents an additional potential problem. Moreover, it has been observed that high doses of conjugate produced vacuoles in cytoplasm of histiocytes, hepatocytes and kidney tubule cells of *Cynomolgus* monkeys (Renoldi et al. 1990) and in hepatic cells of rats and mice (Flume et al. 1992). In *Cynomolgus* monkeys the conjugate has also displayed neuromuscular signs of toxicity (Renoldi et al 1990). Another system developed by Fiume et al. (1986-b) was based in a different carrier constituted by galactosylated poly(L-lysine). Although possesses some advantages over lactosaminated human serum albumin, this system is rather toxic, thus precluding the possibility of its clinical use. With an identical objective, of increasing the therapeutic index of vidarabine, Guise et al. (1990) loaded nanoparticles of polyisohexylcyanoacrylate with ARA-A, but the agent lost its antiviral activity, due to a chemical interaction with the cyanoacrylic monomer, during the polymerization process.

The use of liposomes as drug carriers can promote the concentration of a drug in particular tissues, prolongs drug levels in serum or in tissues, reduces toxicity, and/or enhances the efficacy of the encapsulated drug (Hwang 1987, Sculier 1988, Lopez-Berestein et al. 1989, Perez-Soler 1989, Szoka 1990, Gregoriadis and Florence 1993). Furthermore. the preferential uptake of liposomes by liver cells (Scherphof et al. 1983, Poste et al. 1984). opens some important therapeutic perspectives in the particular case of viral hepatitis. Thus. entrapment of vidarabine in liposomes, by altering biodistribution. may constitute a convenient approach to improve its therapeutic index, promoting an enhancement of drug delivery at the infected sites and avoiding the tissues where the drug produces toxic effects. Moreover, liposomes may protect vidarabine in bloodstream from its rapid deamination by adenosine deaminase, which transforms the drug into 9-β-D-arabinofuranosyl-hypoxantine (ARA-Hx) (Buchanan et al. 1980, Whitley et al. 1980-a and 1980-b). This one is several times less active as an antiviral agent than ARA-A or ARA-AMP (Gephart and Lerner 1981). Entrapment of ARA-A in liposomes have been attempted in the past, but only with limited success as the encapsulation efficiencies of the formulations were very low, ranging from 2.2 to 6.1% (Mayhew et al. 1984).

Accordingly, what is need in the art is a method for preparing liposomal formulations of vidarabine or a vidarabine derivative with high incorporation efficiency and high intraliposomal drug concentration, low relative toxicity to the patient and high pharmacological activity against the virus.

### Summary of the invention

The present invention is addressed to the preparation of stable liposomes with efficient incorporation and high levels of intraliposomal vidarabine (or vidarabine derivative). When administered to animals these formulations are nontoxic and nonimmunogenic, accumulate in a great extension in liver (either in hepatocytes or in Kupffer cells) and spleen, prolong the residence time of the drug in bloodstream and increase its therapeutic index.

The process for the preparation of liposomal formulations is characterized by forming multilamellar liposomes containing the antiviral and subjecting the liposomes to lyophilization, controlled rehydration and, optionally, extrusion under pressure. After preparation, formulations are again subjected to lyophililzation and stored under vacuum at 4°C.

### Description of the specific embodiments

The processes of the present invention provide liposomal formulations of vidarabine (or vidarabine derivative) with high incorporation efficiency and intraliposomal drug concentration. low toxicity and high pharmacological activity.

The present invention comprises a process of dehydration and controlled rehydration, followed by an optional extrusion process through a porous membrane, or other liposome sizing procedure, and a second dehydration. This process comprises the following main steps: a) multilamellar liposomes (MLV) which contain intra and extra-liposomal drug are prepared: b) dehydration by lyophilization of the MLV; c) controlled rehydration using isotonic mannitol solution, physiologic saline solution or another physiologic osmolarity medium; d) dilution of liposomes before optional extrusion using a high dilution volume of isoosmotic medium; e) extrusion using starting material which has not been previously separated from extraliposomal drug; f) separation of extraliposomal drug; g) lyophilization of the final preparation.

According to the present invention, to prepare multilamellar liposomes a chloroform or chloroform-methanol lipid mixture (ranging from about 50 mM up to 100 mM or more) is dried under nitrogen stream. The resulting total lipid concentration ranges from about 10 mM up to about 100 mM. Suitable lipids, hydrogenated or not, for use in the formulations are present individually or in mixtures, in different proportions: cholesterol (CHOL), phosphatidylcholine (PC), hydrogenated phosphatidylcholine (HPC), distearoylphosphatidylcholine (DSPC), sphingomyelin (SM), dioleolylphosphatidylcholine (DOPC), dioleoylphosphatidylglycerol (DOPG), phosphatidylglycerol (PG), dimirystoylphosphatidylcholine (DMPC), dipalmitoylphosphatidylcholine (DPPC), gangliosides, ceramides, phosphatidylinositol (PI), phosphatidic acid (PA), dicetylphosphate (DcP), dimirystoylphosphatidylglycerol (DMPG), stearylamine (SA), dipalmitoylphosphatidylglycerol (DPPG) and any similar synthetic lipids. The lipid mix is preferably charged. The liposomal preparations are typically mixtures of at least two components and more usually three or more: glycerophospholipid (e.g. PC, DMPC, DPPC), CHOL (optionally present) and a charged molecule (lipidic or not), such as PI, PG, SA, or another charged molecule, with each component of the liposomal preparation (when present) in molar ratios of 40-70%, 10-50% and 5-25%, respectively.

Depending on the solubility of the vidarabine derivative used, the drug is dissolved together with the lipids or added to the lipid film in the aqueous hydration medium.

For the encapsulation of a water soluble vidarabine derivative, such as ARA-AMP, the components of the lipid mixture are chosen so at least one component (e.g. phosphatidylinositol, phosphatidylglycerol, stearylamine) is charged and enlarges the aqueous compartments of the liposomes where the water soluble vidarabine derivative is located. For ARA-AMP cationic molecules, such as stearylamine, are most preferable once, besides that effect, they are also capable of establishing electrostatic interactions with the drug. The positively charged molecule is present in the lipid mixture at a concentration of at least 5-25%, preferably at about 17% concentration of total lipid components. For example, a preferred combination is phosphatidylcholine:cholesterol:stearylamine at 10:5:3, with total lipid concentration ranging from 17 to 85 mM or more. The resultant positively charged lipid starting mix produces stable liposomes with high ARA-AMP encapsulation efficiencies and intraliposomal drug concentrations, while minimizing drug-associated toxicity and maximizing antiviral effectiveness. The lipid film is hydrated with an aqueous solution of ARA-AMP. The concentration of the drug solution can vary considerably, from as low as about 0.06 mg/ml up to as much as 6 mg/ml or more, but more typically 1 mg/ml up to 5 mg/ml as described in an example below. The corresponding concentration of lipid for hydration ranges up to17 mM to as high as 85 mM or more. It is desirable to produce liposomes with high drug/lipid ratio, without exceeding saturation of lipidic membrane of liposomes. Going beyond saturation the encapsulation efficiency will be reduced without increasing intraliposomal drug. Thus, it is important that the encapsulation efficiency of the process to be as high as possible to produce liposomes containing adequate amounts of therapeutic drug, even for small initial amount of drug

As noted above, the concentration of vidarabme in the lipid solution or in the aqueous solution used to hydroto the lipid film, and thus in the final liposome, can vary considerably depending on the particular drug chosen. the components of the lipidic mixture, ionic strength, osmolarity. pH, temperature and incorporation method used. Vidarabine or derivatives in the resulting antiviral liposomal formulation can be contained primarily in the aqueous phase, in the lipid phase, or in the aqueous and lipid phases.

Multilamellar liposomes so formed are then subjected to freezing either in liquid nitrogen (-170°C) or in a deep freezer (-70°C). for at least one hour. followed by lyophilization on a freeze dryer (lyophilizer) at 25 mtorr, for at least overnight. The resulting powder is rehydrated in two successive steps: first with a sugar solution of physiologic osmolarity (e g. a 300 mM mannitol or glucose solution), at a portion (e.g. 1/10) of the initial volume, with vigorous vortexing followed by a stabilizing rest of period at room temperature of about 30 min.; secondly the volume is brought up to the initial volume with physiologic saline solution (154 mM NaCl solution) followed by a 10 min. stabilizing period at room temperature. The resulting liposome/drug mixture is then diluted 5 to 10-fold with physiologic saline solution and, Optionally is sized Finally, the non-sized liposomes and the sized ones are ultracentrifuged twice at 250 000 xg for 30 min. or once at 370 000 xg for 45 min., respectively. In both cases pellets are resuspended in appropriate volumes of 10% sucrose or 10% trehalose solutions.

Several techniques are available for sizing liposomes to desired size One sizing method is homogenization. which relies on shearing energy to fragment large liposomes into smaller ones In a typical homogenization procedure, multilamellar vesicles are recirculated through a standard emulsion homogenizer until selected liposome sizes, typically between about 0.1 and 0.5 microns. are observed. Extrusion liposome through a small-pore polycarbonate membrane or an asymmetric ceramic membrane under pressure is also an effective method for reducing liposome sizes to a relatively well-defined size distribution. Typically, the suspension is cycled through the membrane one or more times until the desired liposomes size distribution is achieved. The liposomes may be extruded through successively smaller-pore membranes (e g. from 1.0 µm down to 0.05 µm, as desired) to achieve a gradual reduction in liposome size. In any of the methods the particle size distribution can be monitored by conventional dynamic light scattering.

After the referred steps of hydration of the lipid film, lyophilization, optional extrusion and extraliposomal vidarabine separation, liposomal suspension is frozen at -170°C or -70°C and lyophilized again in the same conditions referred above. Upon reconstitution with sterile deionized water of the lyophilized powders. stored under vacuum at 4°C. non significant changes of the liposome diameters are observed and more than 50% (for water soluble derivatives) or at least 90% (for lipophitic derivatives) of the initially entrapped drug is in the liposomal form.

A particularly preferred embodiment of the invention produces ARA--AMP liposomal formulations with high encapsulation efficiency at a low initial drug:lipid ratio. In this embodiment the lipid mixture is phosphatidylcholine cholesterol:stearylamine at 10:5:3, with lipid concentration of 17-85 mM. Hydration takes place with, e.g., 0.6 to 6.0 mg/ml ARA-AMP, followed by freezing at -170°C or - 70°C and lyophilization. Rehydration to a final osmolarity of 300 mosm/kg is accomplished by the addition of 300 mM mannitol solution in one-tenth the volume of the antiviral solution previously used, followed by filling to the final volume with 154 mM NaCl. Upon dilution in physiologic saline solution and ultracentrifugation as described above, the final liposomal/ARA-AMP formulation has a diameter ranging 0.30 µm to 0.80 µm, but can be smaller and more homogenous if extruded. and possessed an encapsulation efficiency of up to 67.5± 12%, and a drug/lipid ratio of 19-50 g/mol. As with the other preparations described herein the drug is secluded from the external medium (in contrast to typical drug-lipid conjugates) and cannot be displaced from the liposome by a stronger ligand as might occur in other preparations.

Vidarabine (or vidarabine derivative) liposomal formulations prepared according to the processes of the present invention exhibit encapsulation efficiencies of up to 100% without any chemical alteration of the entrapped drug These formulations exhibit high stability either in the lyophilized form or upon reconstitution, are nontoxic and nonimmunogenic, accumulate preferentially in the liver (either in hepatocytes or in Kupffer cells) and spleen and exhibit higher pharmacological activity than the free drug.

The improved therapeutic and pharmacokinetic effects that result are not necessarily related to the final amount of drug in the liposome/drug mixture but the fact that the drug is in a liposomal form having the characteristics described herein

The high encapsulation efficiencies achieved with low drug/lipid ratios indicate that vidarabine (or vidarabine derivative) liposomal formulations of the invention are also highly effective as therapeutic agents. In an *in vivo* animal model of woodchucks chronically infected with woodchuck hepatitis virus (WHV) the formulations prepared by the present process demonstrated an higher therapeutic activity when compared to free drug. This selected animal model is considered by several authors (Summers e: al. 1978; Ponzetto et al. 1991) an ideal model for research on antiviral agents active against HBV. The observed results indicate that the vidarabine (or derivative) liposomal formulations described here increase the therapeutic index of the drug and are highly effective agents in the therapeutic of chronic type B hepatitis.

Vidarabine liposomal formulations of the invention may contain additional substances which serve to target the liposome and hence the antiviral drug to a particular tissue or cell, such as hepatocyte, as well as increase the half-life of the formulation. In these preparations the antiviral drug is incorporated as part of a liposome as described herein, alone, or in conjuction with a targeting molecule, such as galactosyl terminating ligands or monoclonal antibodies, which binds to, e.g., a receptor existent at the hepatocyte surface.

The formulation to be administered will, in any event, contain a quantity of the liposome/vidarabine (or derivative) complex sufficient to achieve the desired therapeutic effect in the subject being treated. The invention provides pharmaceutical formulations for parenteral administration, preferably intravenous, which comprise a liposome/vidarabine (or derivative) complex at a physiological osmolarity, suspended in an acceptable vehicle as desired, preferably an aqueous vehicle (e.g. 0.9% NaCl or 10% sucrose solutions). These formulations may be sterilized by conventional, well known sterilization techniques, or may be sterile filtered. The formulations may contain pharmaceutically acceptable auxiliary substances as required to preserve the quality or to approximate physiological conditions, such as pH adjusting and buffering agents, tonicity adjusting agents. antioxidants, and other adjuvants, for example, sodium acetate, sodium lactate, sodium chloride, potassium chloride, calcium chloride, glucose, a-tocopherol. Actual methods for preparing parenterally administrable formulations will be known or apparent to those skilled in the art and are described in more detail in for example. *Remington's Pharmaceutical Sciences,* 17^{th} ed., Mack Publishing Company, Easton, PA (1985).

Pharmaceutical formulations of the invention may be administered to a warm-blooded animal. such as humans, already suffering from a chronic type B hepatitis infection, during a sufficient period and in a sufficient amount to terminate or significantly inhibit the progression of the infection. Amounts adequate to accomplish these effects are defined as "therapeutically effective doses". Amounts effective for this use will depend on the severity of the infection and the general state of health of the patient being treated. The amounts will generally be less than those typically employed with free drug under similar circumstances. The amount of drug administered via the liposomal/drug formulations of the invention can also be increased above those typically used for free drug, due to the minimization of toxicity to the patient and the overall increased therapeutic effectiveness of the preparations compared to free drug, as might be necessary in the case of severe, life-threatening infections. Determining actual amounts of the liposomal/drug complexes necessary to treat a particular condition as described above will be through standard empirical methods well known in the art.

The following examples of physicochemical and biological analysis made with the vidarabine liposomal formulations prepared by the present processes are offered by way of illustration, not by way of limitation.

### Examples

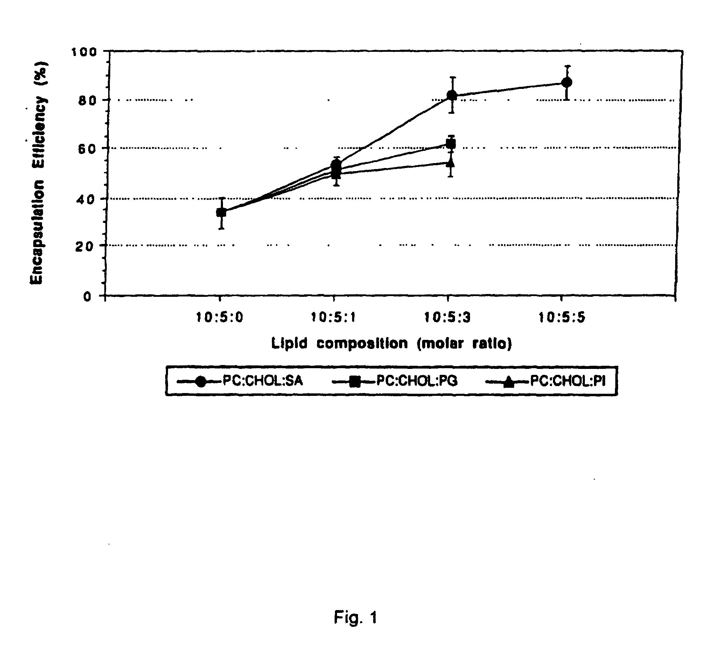

These examples illustrate analysis of vidarabine liposomal formulations prepared as described above, where the vidarabine derivative used was ARA-AMP.
- Encapsulation of vidarabine monophosphate (ARA-AMP) in liposomes
   Table I shows the effect of lipid composition on incorporation of ARA--AMP in liposomes. Formulations were prepared with different lipid compositions in a relative lipid concentration of 17mM and an initial drug/lipid molar ratio of 1/10, using sterile deionized water as rehydration medium. Encapsulation efficiency (E.E.) means the ratio of the final drug/lipid and the initial drug/lipid and is expressed in percentage. The percent recovery indicates the percentage of final to initial drug or lipid concentration. The presence of cholesterol and of charged lipids appeared to influence the encapsulation parameters mentioned above. Cholesterol decreased the ability of ARA-AMP encapsulation (Form. 2), which was promoted, on the contrary, by the inclusion of charged lipids in liposomes (Forms. 3 to 9). This last effect was dependent on the molar fraction of charged lipid present and for negative phospholipids (PI and PG) an almost complete saturation of the vesicles was achieved at 10:5:1 molar ratio (Fig. 1). Positively charged liposomes, obtained by inclusion of SA, exhibited a much higher loading capacity than negative and neutral liposomes. In this case, the encapsulation efficiency was strongly dependent on the SA content, increasing with the increase of SA molar fraction in the liposomes up to 81.6 ± 7.4 % at the 10:5:3 (PC:CHOL:SA) molar ratio. A plateau was reached at this ratio, indicating that vesicle saturation was achieved (Fig. 1). The 10:5:5 molar ratio did not show a significant increase of the ARA-AMP capture efficacy (86.6 ± 6.8 %). In all dispersions were not observed any insoluble non-liposomal lipid-drug precipitates.

**TABLE I**

| Effect of lipid composition on encapsulation parameters of ARA-AMP in liposomes | | | | | | |
|---|---|---|---|---|---|---|
| Formulation n° | Lipid composition (molar ratio) | Di/Li (g/mol) | Df/Lf (g/mol) | Rec. (%) | | E.E. (%) |
| | | | | Lipid | ARA-AMP | |
| 1 | PC | 37.6 ± 4.5 | 19.6 ± 2.8 | 87.0 ± 6.4 | 45.3 ± 3.9 | 52.1 ± 3.1 |
| 2 | PC:CHOL (2:1) | 33.6 ± 3.3 | 11.3 ± 2.0 | 93.6 ± 4.5 | 31.6 ± 4.8 | 33.9 ± 6.6 |
| 3 | PC:CHOL:PI (10:5:1) | 35.2 ± 1.6 | 17.5 ± 2.2 | 92.7 ± 2.5 | 45.8 ± 3.1 | 49.6 ± 4.7 |
| 4 | PC:CHOL:PG (10:5:1) | 35.3 ± 2.2 | 18.0 ± 0.8 | 85.7 ± 1.9 | 44.5 ± 2.3 | 51.2 ± 2.4 |
| 5 | PC:CHOL:SA (10:5:1) | 39.0 ± 2.1 | 20.8 ± 0.1 | 84.6 ± 6.3 | 45.0 ± 1.7 | 53.4 ± 2.9 |
| 6 | PC:CHOL:PI (10:5:3) | 37.1 ± 2.5 | 19.9 ± 1.1 | 85.1 ± 6.5 | 46.1 ± 7.5 | 54.0 ± 5.7 |
| 7 | PC:CHOL:PG (10:5:3) | 34.8 ± 0.4 | 21.5 ± 1.4 | 86.8 ± 5.8 | 53.4 ± 0.9 | 61.7 ± 3.4 |
| 8 | PC:CHOL:SA (10:5:3) | 38.4 ± 4.1 | 31.5 ± 5.8 | 84.6 ± 2.4 | 68.9 ± 5.4 | 81.6 ± 7.4 |
| 9 | PC:CHOL:SA (10:5:5) | 35.5 ± 1.2 | 30.9 ± 3.5 | 84.7 ± 11.7 | 73.3 ± 8.0 | 86.8 ± 6.8 |
| Values listed represent the mean ± SD of at least three preparations. | | | | | | |
| Di/Li: ARA-AMP/lipid initial molar ratio. Df/Lf: ARA-AMP/lipid final molar ratio. | | | | | | |
| Rec: recovery. E.E.: encapsulation efficiency. | | | | | | |

To study the effect of the extrusion process on encapsulation parameters of ARA-AMP in liposomes, a series of experiments was carried out using liposomes prepared as described previously further comprising a step of sizing by extrusion before separation of the extraliposomal drug. Rehydration to a final osmolarity of 300 mosm/kg was accomplished by adding 300 mM mannitol solution in one tenth the volume of the ARA-AMP solution used, followed by filling to the initial volume with 154 mM NaCl solution. Liposomes were extruded through polycarbonate filters of 800 nm (VET₈₀₀) or sequentially through filters of 800 nm, 600 nm and 400 nm (VET₄₀₀). A direct relationship between liposomes size, loading capacity and encapsulation efficiency was observed (Table II).

**TABLE II**

| Effect of extrusion on the size of liposomes, ARA-AMP loading capacity and encapsulation efficiency | | | | | | |
|---|---|---|---|---|---|---|
| Liposome type | Mean diameter (µm) | Poly | Df/Lf (g/mol) | Rec. (%) | | E.E. (%) |
| | | | | Lipid | ARA-AMP | |
| DRV | 0.59 ± 0.18 | 0.36 ± 0.12 | 19.2 ± 3.3 | 95.3 ± 4.0 | 59.2 ± 3.5 | 62.0 ± 1.2 |
| VET₈₀₀ | 0.48 ± 0.13 | 0.17 ± 0.03 | 15.9 ± 0.5 | 84.0 ± 2.0 | 37.3 ± 1.7 | 44.3 ± 1.3 |
| VET400 | 0.30 ± 0.07 | 0.16 ± 0.05 | 13.7 ± 2.8 | 78.6 ± 4.8 | 31.9 ± 3.2 | 40.9 ± 5.7 |
| Values listed represent the mean ± SD of at least three preparations. | | | | | | |
| Di/Li: ARA-AMP/lipid initial molar ratio. Df/Lf: ARA-AMP/lipid final molar ratio. | | | | | | |
| DRV: dehydration-rehydration vesicle. VET: vesicle obtained by extrusion. Rec: recovery. | | | | | | |
| E.E.: encapsulation efficiency. Poly: polydispersity index (is a measure of the liposome homogeneity and ranges from 0.0, for a perfectly homogeneous, to 1.0, for a completely inhomogeneous dispersion). | | | | | | |

- Encapsulation of ARA-AMP *versus* drug/lipid initial ratio

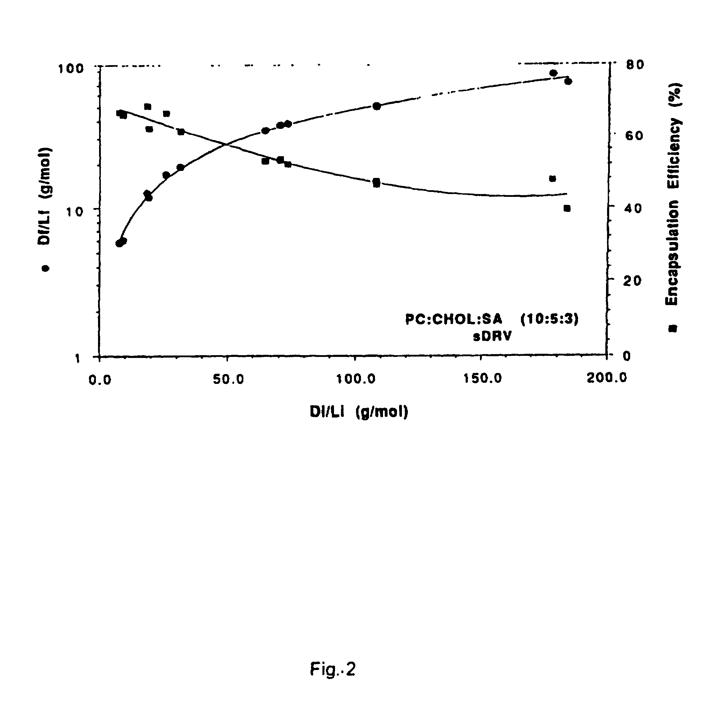

Figs. 2 shows the effect of the initial ARA-AMP/lipid molar ratio on final ARA--AMP/lipid molar ratio and encapsulation efficiency in DRV type liposomes, obtained with 17 mM of total lipid concentration and rehydrating the lyophilized powder with 300 mM mannitol solution in the first step and 154 mM NaCl solution in the second, as already described.

Either for DRV type liposomes or for VET type liposomes, prepared in the same conditions, the amount of incorporated ARA-AMP was increased when initial drug/lipid ratio increased until a plateau was reached. At this point vesicle saturation was achieyed and the correspondent initial and final ARA-AMP/lipid molar ratios were dependent on the lipid composition and the liposome type (Table III). On the contrary, encapsulation efficiency was maximum in all cases for low initial ARA-AMP/lipid molar ratios and increasing this ratio the encapsulation efficiency dropped to smaller values, which also depend on the lipid composition and the liposome type (Table III). The results obtained show that high loading capacities and encapsulation efficiencies are achieved with low drug/lipid molar ratios for these illustrative liposomal formulations prepared by the present process, specially for those including stearylamine.

**TABLE III**

| Initial and final ARA-AMP/lipid molar ratios and encapsulation efficiency correspondent to saturation of the liposomes | | | | |
|---|---|---|---|---|
| Liposome type | Lipid composition (molar ratio) | Di/Li (g/mol) | Df/Lf (g/mol) | E.E. (%) |
| VET₈₀₀ | PC:CHOL:PI (10:5:1) | 74 | 18 | 24 |
| VET₈₀₀ | PC:CHOL:PG (10:5:1) | 71 | 22 | 30 |
| VET₈₀₀ | PC:CHOL:SA (10:5:1) | 71 | 19 | 29 |
| VET₄₀₀ | PC:CHOL:SA (10:5:3) | 170 | 55 | 33 |
| DRV | PC:CHOL:SA (10:5:3) | 185 | 80 | 43 |
| Di/Li: ARA-AMP/lipid initial molar ratio. Df/Lf: ARA-AMP/lipid final molar ratio. | | | | |
| DRV: dehydration-rehydration vesicle. VETx: vesicle obtained by extrusion through polycarbonate filters of "x" nm pore size. Rec: recovery. E.E.: encapsulation efficiency. | | | | |

- Effect of lipid concentration on ARA-AMP encapsulation

In order to optimize the incorporation of ARA-AMP in liposomes, the effect of lipid concentration on the encapsulation of the drug in DRV and VET composed of PC:CHOL:SA (10:5:3) was studied. Rehydration to a final osmolarity of 300 mosm/kg was accomplished by adding 300 mM mannitol solution in one tenth the volume of the ARA-AMP solution used, followed by filling to the initial volume with 154 mM NaCl solution. Encapsulation efficiency can be improved significantly if lipid concentration increases to 85 mM (Table IV).

**TABLE IV**

| Effect of lipid concentration on encapsulation efficiency of ARA-AMP in liposomes^{a} | | | |
|---|---|---|---|
| Lipid concentration (mM) | Liposome type | Df/Lf (g/mol) | E.E. (%) |
| 17 | VET₄₀₀ | 24.6 ± 2.6 | 36.0 ± 1.6 |
| | DRV | 36.5 ± 2.1 | 52.7 ± 0.6 |
| 85 | VET₄₀₀ | 29.0 ± 1.1 | 44.0 ± 1.7 |
| | DRV | 50.0 ± 0.5 | 67.5 ± 0.3 |
| Values listed represent the mean * SD of at least three preparations. E.E.: encapsulation efficiency. Df/Lf: ARA-AMP/lipid final molar ratio. (a) Lipid composition: PC:CHOL:SA (10:5:3); ARA-AMP/lipid initial molar ratio: 2/10 | | | |

- Stability of liposomal vidarabine monophosphate in saline

The stability in saline of a selected ARA-AMP liposomal formulation was studied at 4°C in order to evaluate the behaviour of the vesicles during storage, upon reconstitution. Liposomes of VET₄₀₀ type were prepared with 17 mM of lipid concentration with composition PC:CHOL:SA (10:5:3) and the following aspects were examined: (1) the ocorrence of changes in vesicle size; (2) retention of entrapped contents.

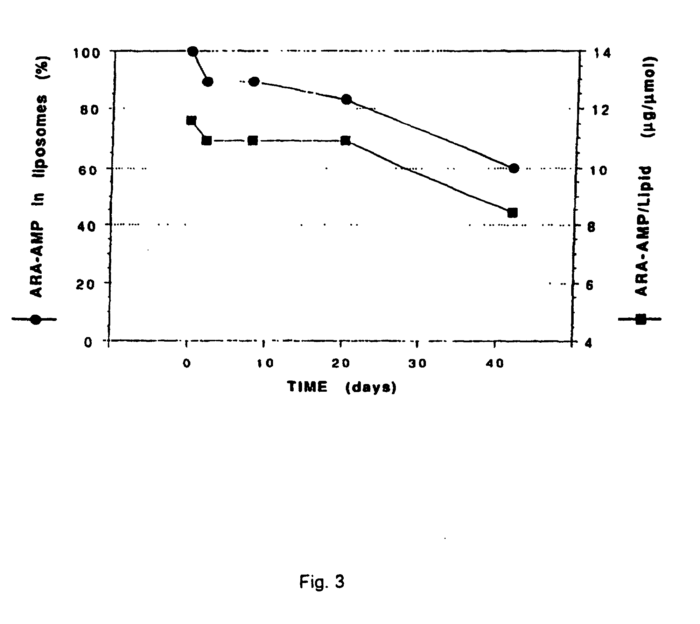

During a 52 days period of storage at 4°C under nitrogen the liposomal formulation did not evidenced significant changes in the mean particle size (Table V). After 20 and 42 days 83% and 60%, respectively, of the initially intraliposomal ARA-AMP was kept in the encapsulated form (Fig. 3).

**TABLE V**

| Stability of liposomal ARA-AMP in saline at 4°C: liposome diameters. | | | |
|---|---|---|---|
| | 0 | TIME (days) 20 | 52 |
| MEAN DIAMETER (µm) | 0.26 ± 0.07 | 0.28 ± 0.03 | 0.28 ± 0.02 |
| Values listed represent the mean ± SD of at least three preparations. | | | |
| VET400. Lipid compostion:PC:CHOL:SA (10:5:3). Initial lipid concentration: 17 mM. | | | |
| ARA-AMP/lipid initial molar ratio: 1/10. | | | |

### Stability of liposomal vidarabine monophosphate in the lyophilized form

The stability of a selected ARA-AMP liposomal formulation under storage in the lyophilized form at 4°C in the vacuum,was studied. Liposomes (VET₄₀₀) of PC:CHOL:SA (10:5:3) lipid composition were obtained as described previously further comprising the step of resuspending the liposomes in 10% sucrose solution. After preparation liposome suspensions were lyophilized. Upon reconstitution with sterile deionlzed water physiologic osmolarity was achieved and non significant changes of the liposome diameters were observed and more than 60 % of the initially entrapped ARA-AMP was in the liposomal form.

**TABLE VI**

| Stability of lyophilized ARA-AMP liposomes^{a} on storage at 4°C under vacuum | | | |
|---|---|---|---|
| TIME (months) | Intraliposomal ARA-AMP Retention (%) | Mean diameter (µm) | Poly^{b} |
| 0 | 100 | 0.32 | 0.14 |
| 0.25 | 85 | 0.33 | 0 14 |
| 0.5 | NM | 0 32 | 0.17 |
| 1 | 63 | 0 30 | 0.17 |
| 1.5 | 68 | 0 28 | 0.10 |
| 4.5 | 63 | 0.31 | 0.13 |
| 6 | 65 | 0.31 | 0.14 |
| 12 | 64 | 0.31 | 0.15 |
| Values listed represent the mean ± SD of at least three preparations. | | | |
| (a) Lipid compostion:PC CHOL SA (10:5:3); ARA-AMP/lipid initial molar ratio: 1/10. | | | |
| (b) Polydispersity index (ranges from 0.0 to 1.0) | | | |
| NM: not measured. | | | |

### In vivo toxicity

Empty liposomes (VET₄₀₀) of different lipid composition (PC.CHOL.PI PC:CHOL:PG; PC:CHOL.SA: all in the 10:5.1 molar ratio) were obtained as described previously and evaluated concerning to toxicity and immunogenicity.

Female *Swiss* mice, 6-7 weeks old at the beginning of the experiment, were injected intraperitonealy with lipid doses of 0.6 mmol/kg of body weight five days a week, during four consecutive weeks. In another study, carried out with identical animals and formulations, liposomal suspensions were injected four times at 3 weeks intervals, being the first injection intraperitoneal and the others intravenous. The injected doses were at least 5 times the correspondent lipid amount used in therapeutic aplications.

Animals were observed either during the administration period or two weeks after the last injection and their aspect (including eventual lesions in the sites of injection) and food and water intake were measured. After this period animals were bleeded and a macroscopic evaluation of the general state of the abdominal organs was performed. Collected sera before and after the injections were assayed for anti-liposomal components IgG, by an microplate ELISA technique, using as antigen either the full composition of each formulation or the individual components. Titters were expressed as the difference between the log₂ of the reciprocal of the antiserum dilution (presenting the same absorvance of the 1/200 dilution of the control serum) and the log₂ of the reciprocal of the 1/200 control serum dilution.

In both injection protocols animals did not evidenced any signs of toxicity and adverse effects. such as anaphylactic shock or dead, were not observed. 1-2 hours after liposomal suspension injection animals injected intravenously appeared less active compared to untreated animals but the same response was observed in mice injected with saline alone. Afterwards injected mice behave completely normally. Only the animals subjected to the first protocol of injections (intraperitoneal) exhibited some granuloms in the abdomen in the sites of injections. These lesions appeared simultaneously (during the thirth week) in the animals injected with the different liposomal suspensions and during the fourth week in mice injected with saline alone. After the sacrifice of animals, organs were find very well individualized without any adderences or alteration in color or in shape. Lesions were circumscribed only to the skin in the injection sites. Food and water intake was very similar in the liposomal injected groups and in untreated group of animals.

All studied animals presented very low titters of antibodies (ranging from 1 to 7), which have been detectable only in 1 of the 5 animals immunized either with SA or PG containing liposomes and in 2 of the 5 animals immunized with PI containing formulation. These results show that liposomes of the studied compositions are practically nonimmunogenic and can be safely used as carriers for nonmacromolecular weight drugs, such as vidarabine, vidarabine monophosphate or any other vidarabine derivative.

In another study carried out in woodchucks, animals were injected intravenously daily for 5 consecutive days with an ARA-AMP liposomal formulation consisting of VET₄₀₀ type liposomes of PC:CHOL:SA (10:5:3) lipid composition. Injected doses ranged from 0.07 to 0.14 mmol/kg, in terms of total lipid and from 2 to 4 mg/kg, in terms of drug. During the treatment and follow-up periods animals behave normally and no adverse effects were noticed.
- Biodistribution of liposomal vidarabine monophosphate
   Biodistribution of a selected ARA-AMP liposomal formulation was studied in *Wistar* rats of 250-350 g of body weight. Liposomes (VET₄₀₀) of lipid composition PC:CHOL:SA (10:5:3) were prepared as described previously using [³H] ARA-AMP. At fixed times (15 min.; 1 hr.; 3 hr.) after the intravenous injection of the radiolabeled liposomes, radioactivities in the blood, liver, spleen and bone marrow were measured. ARA-AMP liposomal formulation affored high residence times of the drug in the bloodstream and a high drug delivery to the liver and spleen, avoiding, simultaneously, the bone marrow. In terms of injected dose, radioactivity in the blood was 32.8 ± 18.9 % at 15 min., decreasing slowly during the time and being 16.2 ± 3.9 % at 3 hours. Accumulation in liver was maximum at 15 min. (21.2 ± 4.6 %). After this time, due to the high metabolic activity of this organ, the accumulated drug amount decreased but remained constant between 1 hour and 3 hours (13.7 ± 6.5 %) due to an equilibrium between the drug delivery and the metabolic activity. Accumulation of ARA-AMP was detected either in hepatocytes or in Kupffer cells. Endotheliat cells did not contribute for the total liver uptake. Accumulation in spleen was lower than in liver and, because of the lower uptake capacity of this organ comparing to the liver, is only maximum at 1 hour (8.8 ± 1.5%) remaining practically constant until 3 hours (7.7 ± 2.2 %). Acummulation in bone marrow was practically null being detectable only 0.5 % of the injected dose at 15 minutes and the same percentage at 3 hours.
- Pharmacological activity of liposomal vidarabine monophosphate
   To evaluate the therapeutic efficacy of a selected vidarabine monophosphate liposomal formulation produced according to the present invention, an animal model of chronic hepatitis was set up using chronically infected woodchucks with woodchuck hepatitis virus (WHV). ARA-AMP liposomal formulation was constituted by VET₄₀₀ type liposomes of lipid composition PC:CHOL:SA (10:5:3).

Animals were injected intravenously with the formulation, as a bolus, for 5 consecutive days at 24 hour intervals. Blood samples were drawn before, during and after the treatment. Bleeding was performed while woodchucks were under anesthesia induced with ketamine hydrochloride. WHV DNA was measured by dot-blot hybridization with ³²P-labeled nick-translated probe of a cloned whole WHV genome (a gift from Dr. J.L. Gerin, Georgetown Univ., Maryland, USA), followed by an autoradiography. Five fold dilutions of all sera from a single treatment were assayed and results were expressed as the reciprocal of the highest serum dilutions giving an autoradiographic signal.

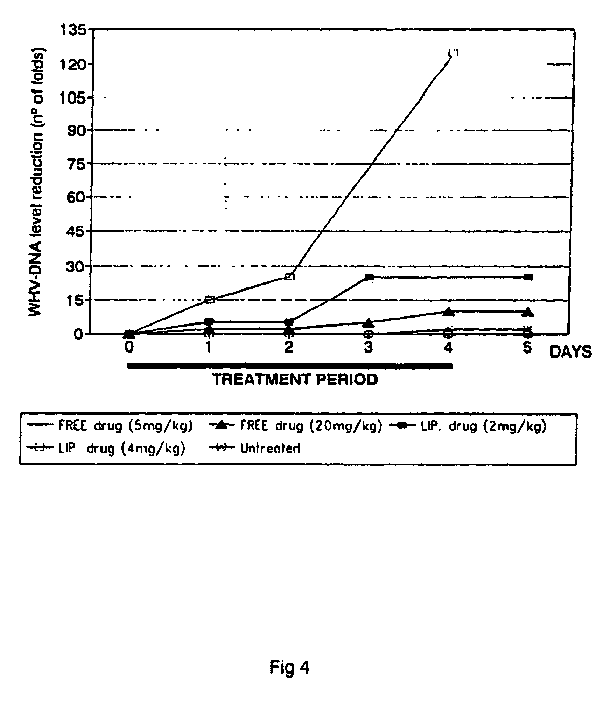

Pharmacological evaluation results showed that antiviral activity of ARA--AMP was greatly improved by liposomal encapsulation. It was observed a clear reduction of WHV-DNA replication after treatment at a significantly lower dose compared to that of the free drug (Fig. 4).

From the results it was concluded that pharmacological activity was greatly increased by vidarabine monophosphate liposomal formulations produced according to the present invention, confirming their usefulness in a clinical setting. Thus, these formulations open a promising approach to circumvent inherent problems associated with the use of vidarabine in the therapy, by means of enhancing the concentration at the sites of action and reducing the therapeutic dose.

### Description of figures

Fig. 1
   Shows the effect of lipid composition in ARA-AMP encapsulation in liposomes (DRV). Error bars indicate SD of at least three preparations. ARA-AMP/lipid initial molar ratio: 1/10. Total lipid concentration: 17 mM. Rehydration medium: deionized water.
Fig. 2
   Shows the variation of encapsulation efficiency and ARA-AMP/Lipid final ratio as function of the drug/lipid initial ratio used. Liposomes (DRV) of PC:CHOL:SA (10:5:3) lipid composition were prepared using a concentration of total lipid of 17 mM.
Fig. 3
   Shows the stability in saline, at 4°C under nitrogen, of a selected ARA-AMP liposomal formulation. composed by liposomes VET₄₀₀ of PC:CHOL:SA (10:5:3) lipid composition.
Fig. 4
   Shows the antiviral activity of free and liposomal ARA-AMP in woodchucks chronically infected with the woodchuck hepatitis virus.

### References

Ayoola, E.A. et al., *Bulletin of the WHO*, 66 (1988) 443-455.

Buchanan, R.A., Kinkel, A.W., et al., *Clin. Pharmacol. Ther*., 27 (1980) 690-696.

Fiume, L., Mattioli, A., et al., *FEBS Lett*., 116 (1980) 185-188.

Fiume, L., Busi, C.,et al., *FEBS* Lett., 129 (1981) 261-264.

Fiume, L., Mattioli, A., et al., *Experientia*, 38 (1982-a) 1087-1089.

Fiume, L., Busi, C., et al., A., *FEBS Lett*., 146 (1982-b) 42-46.

Fiume. L.. Bassi. B.. et al.. *Pharm. Acta Helv*., 60 (1985) 318-320.

Fiume, L., Bassi, B., et al., *Biochem. Pharmacol*., 35 (1986-a) 967-972.

Fiume. L.. Bassi. B.. et al., *FEBS Lett*., 203 (1986-b) 203-206.

Fiume. L.. Busi. C., et al., *Cancer Drug*. *Del*., 4 (1987) 145-150.

Fiume. L.. Bassi. B.. et al., *Pharm. Acta Helv*., 63 (1988-a) 137-139.

Fiume, L., Busi, C., et al., *CRC Crit. Rev. Ther. Drug Carrier Syst*., 4 (1988-b) 265-284.

Fiume, L., Bonino, F., et al., *Lancet*, 2 (1988-c) 13-15.

Fiume, L., Betts. C.M., et al., *J*. *Hepatol*., 15 (1992) 314-322.

Gerin. J.L. *Cur. Opin. Inf. Dis*., 5 (1992) 806-810.

Gephart. G.F. and Lerner, A.M., *Antimicrob. Agents Chemother*., 19 (1981) 170-178.

Gregoriadis. G. and Florence; A.T.. *Drugs,* 45 (1993) 15-28.

Guardia. J., Esteban. R., et al., *Liver,* 6 (1986) 118-122.

Guillevin. L., Lhote. F., et al., *J*. *Rheumatol.,* 20 (1993) 289-298.

Guise, V., Drouin, J.-Y., et al., *Pharm. Res*., 7 (1990) 736-741.

Hwang, K.J., Liposome pharmacokinetics. In Ostro, M.J. (Ed.), *Liposomes: from biophysics to therapeutics*, Marcel Dekker, Inc., New York, 1987, pp. 109-156.

Kanterewick. E., Bruguera, M., et al., *J*. *Clin. Gastroenterol*., 12 (1990) 90-92.

LePage, G.A.. Lin, T.-T., et al., *Cancer Res*., 32, (1972) 2441-2444.

Lok, A.. *JAMA*. 11 (1990) 713.

Lopez-Berestein, G., Bodey. G., et al., *Arch. Intern. Med*., 149 (1989) 2533-2536.

Lunel-Fabiani. F.. Rev. *Prat*., *41* (1991) 315-323.

Marcellin. P., Ouzan, D., et al., *Hepatology*, 10 (1989) 328-331.

Marcellin, P., *Rev*. *Prat., 41* (1991) 1149-1155.

Mayhew, E., Lazo, R. and Vail W.J., Preparation of liposomes entrapping cancer chemotherapeutic agents for experimental *in vivo* and *in vitro* studies. In Gregoriadis, G. (Ed.), *Liposome Technology,* Vol. II, CRC Press, Boca Raton, 1984, pp. 19-31.

Perez-Soler, R., *Cancer Treat. Rev*., 16 (1989) 67-82.

Ponzetto, A., Fiume, L., et al., *Hepatology*, 14 (1991) 16-24.

Posts, G., Kirsh, R. and Koestler, T., The challenge of liposome targeting in vivo. In Gregoriadis, G. (Ed.), *Liposome Technology,* Vol.III, CRC Press Inc., Florida, 1984, pp. 1-28.

Renoldi, A., Maraschin, R., et al., 4-Week intravenous toxicity study in *Cynomolgus* monkeys treated with L-HSA-ara-AMP. RBM, Istituto di Ricerche Biomedich Antoine Marxer, Ivrea, Italy (1990) Exp. n° 890520.

Sacks. S.L., Scullard, G.H., et al., *Antim. Ag. Chemother*., 21 (1982) 93-100.

Schabel Jr., F.M., *Chemotherapy,* 13 (1968) 321-338.

Sculier, J.-P., Coune, A., et al., *Eur. J. Cancer Clin. Oncol*., 24 (1988) 527-538.

Scullard, G.H., Pollard, R.B., et al., *J. Infect. Dis*., 143 (1981) 772-783.

Shannon, W.M., Adenine arabinoside: antiviral activity *in vitro*. In Pavan-Langston, D., Buchanan, R.A. and Alford, C.A.Jr. (Eds.), *Adenine arabinoside: an antiviral agent,* Raven Press. New York. 1975, pp. 1-43.

Sherphof. G.L.. Roerdink, F., et al., *Biol. Cell*., 47 (1983) 47-58.

Smith, C.I., Kitchen, L.W., et al., *JAMA*, 247 (1982) 2261-2265.

Szoka Jr.. F.C., *Biotech. Applied Biochem*., 12 (1990) 496-500.

Tiollais, P.. Dejean, A., et al., Méd./*Sci*, 6 (1990) 96-97.

Thomas, H.C.. Treatment of hepatitis B viral infection. In Zuckerman, A.J. (Ed.), *Viral hepatitis and liver* disease. Alan R. Liss, New York, 1988, pp. 817-822.

Trepo. C., Ouzan. D., et al., *J*. *Hepatol*., 3 (Suppl. 2) (1986) S97-S105.

Weller, I.V.D., Lok. A.S.F., et al., Gut, 26 (1985) 745-751.

Weller. I.V.D., *J*. *Hepatol*.. 3 (Suppl. 2) (1986) S107-110.

Whitley, R.. Alford. C., et al., *Drugs,* 20 (1980-a) 267-282.

Whitley, R.J.. Tucker. B.C., et al., *Antimicrob. Ag. Chemother*., 18 (1980-b) 709-715.

Yokosuka. O.. Omata, M., et al., *Gastroenterology*, 89 (1985) 246-251.

## Claims

1. A process for preparing vidarabine (or vidarabine derivative) liposomal formulations suitable for administration to a mammal which comprises:
forming a multliamellar liposome vidarabine (or derivative) formulation from an aqueous solution and a lipid film mixture containing or not a component having an electric charge:
lyophilizing the liposomal vidarabine (or derivative);
rehydrating the lyophilized liposomal vidarabine (or derivative) to a final physiologic osmolarity first in a non-saline solution of approximately physiologic osmolarity; and
further rehydrating the liposomal vidarabine (or derivative) with a physiologically acceptable saline solution.

2. The process according to claim 1, further comprising the step of sizing the rehydrated vidarabine (or vidarabine derivative) liposomal formulation by extrusion through a porous membrane

3. The process according to claim 2, wherein the vidarabine (or vidarabine derivative) liposomal formulation is extruded under pressure through 0.8 µm membrane.

4. The process according to claim 2, wherein the vidarabine (or vidarabine derivative) liposomal formulation is extruded under pressure sequentially through 0.8 µm 0.6 µm and 0.4 µm membranes.

5. The process according to claim 1, wherein the resulting vidarabine (or vidarabine derivative) liposomal formulation is approximately 0.3 µm - 5.0 µm in diameter.

6. The process according to claim 3, wherein the resulting vidarabine (or vidarabine derivative) liposomal formulation is approximately 0 3 µm - 0 7 µm in diameter.

7. The process according to claim 4, wherein the resulting vidarabine (or vidarabine derivative) liposomal formulation is approximately 0.2 µm - 0.4 µm in diameter.

8. The process according to claims 3 and 4, in which any vidarabine (or vidarabine derivative) not incorporated into the liposomes is removed before *cr* after the extrusion step.

9. The process according to claim 1. in which the concentration of the lipid mixture in the lipidic film is 10 mM up to 100 mM.

10. The process according to claim 9, in which the concentration of the lipid mixture in the lipidic film is 17 mM up to 85 mM.

11. The process according to claim 1, in which a lipid component of the lipidic film mixture having a charged molecule.

12. The process according to claim 11, in which a lipid component of the lipidic film mixture having a positive charge and which is capable of interacting with vidarabine (or derivative) molecule.

13. The process according to claim 11, in which a lipid component of the lipidic film mixture having a negative charge and which is capable of interacting with vidarabine (or derivative) molecule.

14. The process according to claim 12, in which for the encapsulation of vidarabine monophosphate (ARA-AMP) the concentration of the positive molecule is at least 5%.

15. The process according to claim 14. in which for the encapsulation of vidarabine monophosphate (ARA-AMP) the concentration of the positive molecule is approximately 17%.

16. The process according to claim 1, in which the lipidic film mixture is comprised of, in a molar ratio ranging from 0.01 to 10: cholesterol (CHOL), phosphatidylcholine (PC), hydrogenated phosphatidylcholine (HPC), distearoylphosphatidylcholine (DSPC), sphingomyelin (SM), dioleolylphosphatidylcholine (DOPC), dioleoylphosphatidylglycerol (DOPG), phosphatidylglycerol (PG), dimirystoylphosphatidylcholine (DMPC), dipalmitoylphosphatidylcholine (DPPC), gangliosides. ceramides. phosphatidylinositol (PI), phosphatidic acid (PA), dicetylphosphate (DcP). dimirystoylphosphatidylglycerol (DMPG), stearylamine (SA). dipalmitoylphosphatidylglycerol (DPPG) and any other synthetic lipid.

17. The process of claim 16, in which the lipidic film mixture is comprised of a glycerophospholipid selected from phosphatidylcholine, dimirystoylphosphatidylcholine. dipalmitoylphosphatidylcholine; a sterol, such as cholesterol. optionally present: and a charged molecule lipidic or not (phosphatidylinositol, phosphatidylglycerol, stearylamine); with each component of the liposomal preparation when present in molar relative proportions of 40-70%, 10-50% and 5-25%, respectively.

18. The process according to claim 17, wherein, for the encapsulation of vidarabine monophosphate (ARA-AMP), the lipid components of phosphatidylcholine: cholesterol:stearylamine are in a molar ratio of approximately 10:5:3.

19. The process according to claim 1, wherein the lyophilized liposomal vidarabine (or derivative) is rehydrated in a solution of dextrose, galactose, mannitol, sucrose or other sugar at sufficient concentration to maintain a physiologic osmolarity.

20. The process according to claim 1, wherein the physiologically acceptable saline solution of the final rehydration step is the 0.9% NaCl solution.

21. The process according to claim 1, wherein the aqueous solution used to form the multilamellar liposome vidarabine (or derivative) formulation contains a concentration of vidarabine, vidarabine monophosphate or another vidarabine derivative from 0.06 mg/ml to 6 mg/ml.

22. The process according to claim 1, wherein the lipid film includees a derivative of vidarabine

23. The process according to claim 21, wherein the vidarabine derivative is vidarabine monophosphate (ARA-AMP).

24. The process according to claim 21, 22 and 23, **characterized by** producing vidarabine (or vidarabine derivative) liposomal formulations with an encapsulation efficiency up to 100 %.

25. The process according to claim 24, **characterized by** producing vidarabine (or vidarabine derivative) liposomal formulations that exhibit a stability in saline at 4°C higher than 50% and maintain the liposome characteristics after 45 days of incubation.

26. The process according to claims 1,2 and 8, further comprising the step of resuspending the liposomes in 10% sucrose or 10% trehalose solutions or another isotonic sugar solution after remotion of the extraliposomal drug.

27. The process according to claim 26 further comprising the step of lyophilization of the liposomal suspension.

28. The process according to claim 27, **characterized by** producing liposomal formulations that exhibit high stability in the lyophilized state in terms of liposome morphology and intraliposomal drug retention.

29. The process according to claim 28, **characterized by** producing liposomal formulations that, upon reconstitution with sterile deionized water of lyophilized powder, stored under vacuum at 4°C for 1 year non significant changes of the liposome diameters are observed and more than 50% (for water soluble vidarabine derivatives) or at least 90% (for lipophilic derivatives) of the initially entrapped drug is in the liposomal form.

30. The process according to claim 23, in which the ARA-AMP liposomal formulations, when administered to a patient in nontoxic and nonimmunogenic.

31. The process according to claim 23, in which the ARA-AMP liposomal formulations, when injected intravenously to a patient, prolong the residence time of the drug in the bloodstream.

32. The process according to claim 23, in which the ARA-AMP liposomal formulations, when injected intravenously to a patient, accumulate mainly in liver (in a great extension) and spleen.

33. The process according to claim 23, in which the ARA-AMP liposomal formulations, when injected intravenously to a patient, accumulate in hepatocytes and Kupffer cells.

34. The process according to claim 23, **characterized by** producing ARA-AMP liposomal formulations with increased therapeutic index compared to free drug.

35. The process according to claim 23 **characterized by** producing ARA-AMP liposomal formulations with enhanced therapeutic activity, when used in the treatment of type B chronic hepatitis or in other diseases in which they are therapeutically active, compared to free drug.

36. A liposomal formulation suitable for administration to a mammal prepared according to any one of claims 1-35, which comprises vidarabine, vidarabine monophosphate or another vidarabine derivative **characterized by** having a drug concentration from 0.06 mg/ml to 6 mg/ml and a lipid concentration from 17 mM to 85 mM.

37. The vidarabine (or vidarabine derivative) liposomal formulation according to claim 36, wherein the liposomes range from 0.01 µm to 5.0 µm in diameter.

38. The vidarabine (or vidarabine derivative) liposomal formulation of claim 36, wherein the lipid component is comprised of, in a molar ratio ranging from 0.01 to 10: cholesterol (CHOL), phosphatidylcholine (PC), hydrogenated phosphatidylcholine (HPC), distearoylphosphatidylcholine (DSPC), sphingomyelin (SM), dioleolylphosphatidylcholine (DOPC), dioleoylphosphatidylcholine (DOPG), phosphatidylglycerol (PG), dimirystoylphosphatidylcholine (DMPC), dipalmitoylphosphatidylcholine (DPPC), gangliosides, ceramides, phosphatidylinositol (PI), phosphatidic acid (PA), dicetylphosphate (DcP), dimirystoylphosphatidylglycerol (DMPG), stearylamine (SA), dipalmitoylphosphatidylglycerol (DPPG) and any other synthetic lipid.

39. The vidarabine (or vidarabine derivative) liposomal formulation of claim 36, wherein the lipid component is comprised of a glycerophospholipid selected from phosphatidylcholine, dimiristoyIphosphatidyIcholine, dipalmitoylphosphatidylcholine; a sterol, such as cholesterol, optionally present; and a charged molecule lipidic or not (phosphatidylinositol, phosphatidylglycerol, stearylamine); with each component of the liposomal preparation when present in molar proportions of 40-70%, 10-50% and 5-25%, respectively.

40. The liposomal formulation of claim 39, wherein, for the encapsulation of vidarabine monophosphate (ARA-AMP), the lipid component is comprised of phosphatidylcholine:cholesterol:stearylamine in molar relative proportions of 40-70%, 10-50% and 5-25%, respectively.

41. The liposomal formulation of claim 40, wherein, for the encapsulation of vidarabine monophosphate (ARA-AMP), the lipid components of phosphatidylcholine: cholesterol:stearylamine are in a molar ratio of approximately 10:5:3.

42. The liposomal formulation of claim 40, wherein the concentration of the positive molecule (in terms of molar relative lipid proportions) is at least 15%.

43. The vidarabine (or vidarabine derivative) liposomal formulation of claim 36, having approximately physiological osmolarity.

44. The vidarabine liposomal formulation according to claim 36, in which the vidarabine derivative is vidarabine monophosphate (ARA-AMP).

45. The vidarabine (or vidarabine derivative) liposomal formulation according to claims 36-44, which is nontoxic and nonimmunogenic.

46. The vidarabine (or vidarabine derivative) liposomal formulation according to claims 36-44. **characterized by** an increased residence time in bloodstream, compared to free drug.

47. The vidarabine (or vidarabine derivative) liposomal formulation according to claims 36-44, **characterized by** accumulation mainly in liver (in a great extension) and in spleen.

48. The vidarabine (or vidarabine derivative) liposomal formulation according to claims 36-44, **characterized by** an high accumulation of drug in the liver and spleen compared to free drug.

49. The vidarabine (or vidarabine derivative) liposomal formulation according to claims 36-44, **characterized by** producing lower levels of drug in bone marrow compared to free drug.

50. The vidarabine (or vidarabine derivative) liposomal formulation according to claims 36-44. **characterized by** accumulation in hepatocytes and Kupffer cells.

51. The vidarabine (or vidarabine derivative) liposomal formulation according to claims 36-44, **characterized by** an high accumulation of drug in the liver cells compared to free drug.

52. The vidarabine (or vidarabine derivative) liposomal formulation according to claims 36-44, **characterized by** having an increased therapeutic index compared to free drug.

53. The vidarabine (or vidarabine derivative) liposomalformulation according to claims 36-44, **characterized by** having an enhanced therapeutic activity, when used in the treatment of type B chronic hepatitis or in other diseases in which they are therapeutically active, compared to free drug.

54. Utilisation of liposomal formulations according to any one of claims 36-53, for the manufacture of a medicament for a new medical treatment, wherein a therapeutically effective amount of a vidarabine (or vidarabine derivative) liposomal formulation os used.

## Patentansprüche

1. Verfahren zur Herstellung von Liposomenformulierungen aus Vidarabin (oder ein Vidarabinderivat), die geeignet sind für Verabreichung zu einem Säugetieren, umfassend:
ein mehrschichtig Liposomenformulierung aus Vidarabin (oder ein Derivat davon) aus ein wäßrig Lösung und ein Lipidschichtgemisch, ein Bestandteil mit elektrisch Ladung enthaltend oder nicht, herstellen;
die Liposomvidarabin (oder Derivat davon) gefriertrocknen;
die gefriertrocknet Liposomvidarabin (oder ein Derivat davon) zu einem schließlich physiologische Osmolarität, erst in ein nicht salzhaltig Lösung von etwa physiologische Osmolarität rehydratisieren; und
die Liposomvidarabin (oder Derivat davon) mit eine physiologische verträgliche salzhaltig Lösung wieder rehydratisieren.

2. Verfahren nach Anspruch 1, weiter umfassend der Schritt von die rehydratisiert Liposomenformulierung aus Vidarabin (oder ein Vidarabinderivat) durch Extrusion über eine durchlässige Membrane herabsetzen.

3. Verfahren nach Anspruch 2, wobei die Liposomenformulierung aus Vidarabin (oder ein Vidarabinderivat) unter Druck durch eine 0,8 µm Membrane extrudiert wird.

4. Verfahren nach Anspruch 2, wobei die Liposomenformulierung aus Vidarabin (oder ein Vidarabinderivat) unter Druck durch 0,8 µm, 0,6 µm und 0,4 µm Membranen nacheinander extrudiert wird.

5. Verfahren nach Anspruch 1, wobei die erhaltend Liposomenformulierung aus Vidarabin (oder ein Vidarabinderivat) ein Durchmesser zwischen etwa 0,3 µm und 5,0 µm hat.

6. Verfahren nach Anspruch 3, wobei die erhaltend Liposomenformulierung aus Vidarabin (oder ein Vidarabinderivat) ein Durchmesser zwischen etwa 0,3 µm und 0,7 µm hat.

7. Verfahren nach Anspruch 4, wobei die erhaltend Liposomenformulierung aus Vidarabin (oder ein Vidarabinderivat) ein Durchmesser zwischen etwa 0,2 µm und 0,4 µm hat.

8. Verfahren nach Ansprüche 3 und 4, wobei die in die Liposomen nicht aufgenommen Vidarabin (oder ein Vidarabinderivat) vor oder nach der Extrusionschritt ganz entfernt ist.

9. Verfahren nach Anspruch 1, wobei die Konzentration der Lipidgemisch in der lipidisch Film 10 mM bis zu 100 mM ist.

10. Verfahren nach Anspruch 9, wobei die Konzentration der Lipidgemisch in der lipidisch Film 17 mM bis zu 85 mM ist.

11. Verfahren nach Anspruch 1, wobei ein lipidisch Bestandteil von der lipidisch Filmgemisch ein geladen Molekül hat.

12. Verfahren nach Anspruch 11, wobei ein lipidisch Bestandteil der lipidisch Filmgemisch ein positiv Ladung hat und einwirken auf Vidarabin (oder ein Derivat davon) kann.

13. Verfahren nach Anspruch 11, wobei ein Lipidbestandteil der lipidisch Filmgemisch ein negativ Ladung hat und einwirken auf Vidarabin (oder ein Derivat davon) kann.

14. Verfahren nach Anspruch 12, wobei für Verkapselung der Vidarabinmonophosphat (ARA-AMP) der Konzentration der positiv Molekül mindestens 6% ist.

15. Verfahren nach Anspruch 14, wobei für Verkapselung der Vidarabinmonophosphat (ARA-AMP) die Konzentration der positiv Molekül ca. 17% ist.

16. Verfahren nach Anspruch 1, wobei die lipidisch Filmgemisch, in ein Verhältnis zwischen 0,01 bis 10: Cholesterol (CHOL), Phosphatidylcholin (PC), hydriert Phosphatidylcholin (HPC), Distearoylphosphatidylcholin (DSPC), Sphingomyelin (SM), Dioleolylphosphatidylcholin (DOPC), Dioleoylphosphatidylglycerol (DOPG), Phosphatidylglycerol (PG), Dimirystoylphosphatidylcholin (DMPC), Dipalmitoylphosphatidylcholin (DPPC), Ganglioside, Ceramide, Phosphatidylinositol (PI), Phosphatidinsäure (PA), Dicetylphosphat (DcP), Dimirystoylphosphatidylglycerol (DMPG), Stearylamin (SA), Dipalmitoylphosphatidylglycerol (DPPG) und jeder beliebiger synthetisch Lipid einschliesst.

17. Verfahren nach Anspruch 16, wobei die lipidisch Filmgemisch ein Glycerophospholipid gewählt aus der Gruppe Phosphatidylcholin, Dimirystoylphosphatidylcholin, Dipalmitoylphosphatidylcholin einschliesst; wobei ein Sterol, wie Cholesterol ist gegebenenfalls vorliegend; und ein lipidisch oder nicht lipidisch geladen Molekül (Phosphatidylinositol, Phosphatidylglycerol, Stearylamin); mit jeder Bestandteil der Liposomenpräparat wenn vorliegende in ein relativ Verhältnis von 40-70%, 10-50% bzw. 5-25%.

18. Verfahren nach Anspruch 17, wobei für Verkapselung der Vidarabinmonophosphat (ARA-AMP) die lipidische Bestandteile aus Phosphatidylcholin:Cholesterol:Stearylamin ein Molverhältnis von etwa 10:5:3 aufweisen.

19. Verfahren nach Anspruch 1, wobei die gefriertrocknet Liposomenvidarabin (oder ein Derivat davon) rehidratisiert in ein Dextrose-, Galactose-, Manit-, Sucrose- oder ander Zuckerlösung wird, in eine Konzentration genügend für eine physiologische Osmolarität erzuhalten.

20. Verfahren nach Anspruch 1, wobei die physiologische verträgliche Salzlösung in der schließlich rehidratisierung Schritt ein NaCl 0,9% Lösung ist.

21. Verfahren nach Anspruch 1, wobei die wäßrige Lösung daß für Herstellung die mehrschichtig Liposomenformulierungen aus Vidarabin (oder ein Derivat davon) eingesetzt wird eine Konzentration aus Vidarabin, Vidarabinmonophosphat oder einander Vidarabinderivat von 0,06 mg/ml bis 8 mg/ml enthält.

22. Verfahren nach Anspruch 1, wobei der Lipidfilm ein Vidarabinderivat einschliesst.

23. Verfahren nach Anspruch 21, wobei der Vidarabinderivat Vidarabinmonophosphat (ARA-AMP) ist.

24. Verfahren nach Anspruch 21, 22 und 23, **dadurch gekennzeichnet daß** Liposomenformulierungen aus Vidarabin (oder ein Vidarabinderivat) mit ein Verkapselungwirksamkeit bis zu 100% hergestellt sind.

25. Verfahren nach Anspruch 24, **dadurch gekennzeichnet daß** Liposomenformulierungen aus Vidarabin (oder ein Vidarabinderivat) die ein Stabilität in Salzlösung bei 4°C höher als 50% aufweisen und die Eigenschaften der Liposomen nach 45 Tagen Inkubationszeit erhalten hergestellt werden.

26. Verfahren nach Ansprüche 1, 2 und 8, einschlißend noch die Liposomen in 10% Sucrose oder 10% Trehalose Lösungen oder in eine isotonisch Lösung von anderen Zucker nach entfernen der extraliposomale Wirkstoff wieder in Suspension bringen.

27. Verfahren nach Anspruch 26 einschlißend noch wieder der Schritt von Gefriertrocknung der Liposomensuspension.

28. Verfahren nach Anspruch 27, **gekennzeichnet durch** die Vorbereitung von Liposomenformulierungen, mit eine hohe Stabilität unter gefriertrocknen Form in Beziehung auf die Morphologie und intraliposomale Zurückbehaltung der Arzneimittel.

29. Verfahren nach Anspruch 28, **gekennzeichnet durch** die Vorbereitung von Liposomenformulierungen, die nach Wiederherstellung mit sterile, entionisierte Wasser des gefriertrocknes Pulvers, daß 1 Jahr unter Vakuum, bei 4°C gelagert ist, keine bedeutungsvolle Wandlungen des Liposomendurchmesser beobachtet wird und mehr als 50% (für wasserlösliche Vidarabinederivate) oder mindestens 90% (für lypophilische Derivate) von den anfangs gefangenen Wirkstoff in liposomische Form ist.

30. Verfahren nach Anspruch 23, wobei die zu ein Patient verabreicht Liposomenformulierungen ARA-AMP nichtgiftig und nichtimmunogenisch ist.

31. Verfahren nach Anspruch 23, wo die ARA-AMP Liposomenformulierungen der Verweilzeit der Wirkstoff in Blutkreislauf verlängert, wenn sie dem Patient i.v. injeziert wird.

32. Verfahren nach Anspruch 23, wo die ARA-AMP Liposomenformulierungen sich hauptsächlich in die Leber (in großen Umfang) und Milz sammeln, wenn sie dem Patient i.v. injeziert wird.

33. Verfahren nach Anspruch 23, wo die Liposomenformulierungen ARA-AMP sich in Hepatozyten und Kuppfer Zelle sammeln, wenn sie dem Patient i.v. injeziert wird.

34. Verfahren nach Anspruch 23, **gekennzeichnet durch** die Herstellung von Liposomenformulierungen ARA-AMP mit einem erhöhten therapeutisch Zahl im Vergleichung mit der frei Wirkstoff.

35. Verfahren nach Anspruch 23, **gekennzeichnet durch** die Herstellung von Liposomenformulierungen ARA-AMP mit einem erhöhten therapeutischen Wirkung wenn sie zur Behandlung von chronisch B Hepatitis oder andere Krankheiten, wo sie therapeutische Wirkend sind, verwendet werden im Vergleichung mit der frei Wirkstoff

36. Liposomenformulierung, die für die Verabreichung am Säugetier geeignet ist, hergestellt nach einem der Ansprüche 1-35, umfassend Vidarabin, Vidarabinmonophosphate oder andere Vidarabinderivat, **gekennzeichnet durch** eine Wirkstoffkonzentration zwischen 0,06 mg/ml und 6 mg/ml und eine Lipidkonzentration zwischen 17 mM und 85 mM.

37. Vidarabin Liposomenformulierung (oder ein Vidarabinderivat) nach Anspruch 36, wobei die Liposomen ein Durchmesser zwischen 0,01 µm und 5,0 µm aufweisen.

38. Liposomenformulierungen aus Vidarabin (oder ein Vidarabinderivat) nach Anspruch 36, wobei die Lipidbestandteil, in ein Verhältnis zwischen 0,01 bis 10: Cholesterol (CHOL), Phosphatidylcholin (PC), hydriert Phosphatidylcholin (HPC), Distearoylphosphatidylcholin (DSPC), Sphingomyelin (SM), Dioleolylphosphatidyicholin (DOPC), Dioleoylphosphatidylcholin (DOPG), Phosphatidylglycerol (PG), Dimirystoylphosphatidylcholin (DMPC), Dipalitoylphosphatidylcholin (DPPC), Ganglioside, Ceramide, Phosphatidylinositol (PI), Phosphatidinsäure (PA), Dicetylphosphat (DcP), Dimirystoylphosphatidylglycerol (DMPG), Stearylamin (SA), Dipalmitoylphosphatidylglycerol (DPPG) und jede beliebig synthetisch Lipid darstellt.

39. Liposomenformulierung aus Vidarabin (oder ein Vidarabinderivat) nach Anspruch 36, wobei die Lipidbestandteil einschliesst ein Glycerophospholipid ausgewählt aus Phosphatidylcholin, Dimiristoylphosphatidylcholin, Dipalmitoylphosphatidylcholin; ein Sterol wie Cholesterol, gegebenenfalls vorliegend; und eine lipidische oder nichtlipidische geladene Molekül (Phosphatidylinositol, Phosphatidylglycerol, Stearylamin), worin jeder Bestandteil aus der Liposomenvorbereitung, wenn vorliegend, eine relative Verhältnis zwischen 40-70%, 10-50% bzw. 5-25% beträgt.

40. Liposomenformulierung nach Anspruch 39, wobei der lipidiche Bestandteil für die Verkapselung der Vidarabinmonophosphat (ARA-AMP) Phosphatidylcholin:Cholesterol:Stearylamin in eine relative Verhältnis zwischen 40-70%, 10-50% bzw. 5-25% einschliesst.

41. Liposomenformulierung nach Anspruch 40, wobei für Verkapselung der Vidarabinmonophosphat (ARA-AMP) die lipidische Bestandteilen aus Phosphatidylcholin: Cholesterol:Stearylamin in ein Molverhältnis von etwa 10:5:3 vorhanden sind.

42. Liposomenformulierungen nach Anspruch 40, wobei die Konzentration der positiv Molekül (als relativ Lipid Molverhältnis) mindestens 15% beträgt.

43. Liposomenformulierungen aus Vidarabin (oder ein Vidarabinderivat) nach Anspruch 36, daß nahezu physiologische Osmolarität hat.

44. Liposomenformulierungen aus Vidarabin (oder ein Vidarabinderivat) nach Anspruch 36, wobei der Vidarabinderivat Vidarabinmonophosphat (ARA-AMP) ist.

45. Liposomenformulierungen aus Vidarabin (oder ein Vidarabinderivat) nach Ansprüche 36-44, daß nichtgiftig und nichtimmunogenisch ist.

46. Liposomenformulierungen aus Vidarabin (oder ein Vidarabinderivat) nach Ansprüche 36-44, **dadurch gekennzeichnet daß** sie ein erhöhten Verweilenzeit ins Blutkreislauf in Vergleich zu die freie Wirkstoff aufweist.

47. Liposomenformulierungen aus Vidarabin (oder ein Vidarabinderivat) nach Ansprüche 36-44, **dadurch gekennzeichnet daß** sie hauptsächlich in die Leber (in großen Umfang) und Milz sich ansammelt.

48. Liposomenformulierungen aus Vidarabin (oder ein Vidarabinderivat) nach Ansprüche 36-44, **dadurch gekennzeichnet daß** die Wirkstoff in die Leber und Milz sich ansammelt in höher Menge als die freie Wirkstoff.

49. Liposomenformulierungen aus Vidarabin (oder ein Vidarabinderivat) nach Ansprüche 36-44, **dadurch gekennzeichnet daß** sie niedrig Gehalt der Wirkstoffen ins Knochenmark erzeugt als die freie Wirkstoff.

50. Liposomenformulierungen aus Vidarabin (oder ein Vidarabinderivat) nach Ansprüche 36-44, **dadurch gekennzeichnet daß** sie sich in Hepatozyten und Kupffer Zelle sammeln.

51. Liposomenformulierungen aus Vidarabin (oder ein Vidarabinderivat) nach Ansprüche 36-44, **dadurch gekennzeichnet daß** sie in höher Menge in Leberzellen ansammelt als die freie Wirkstoff.

52. Liposomenformulierungen aus Vidarabin (oder ein Vidarabinderivat) nach Ansprüche 36-44, **dadurch gekennzeichnet daß** sie ein erhöhten Therapeutikzahl als die freie Wirkstoff hat.

53. Liposomenformulierungen aus Vidarabin (oder ein Vidarabinderivat) nach Ansprüche 36-44, **dadurch gekennzeichnet daß** sie ein verbessert therapeutisch Wirkung zur Behandlung von Typ B chronisch Hepatitis oder von anderen Erkrankungen für denen sie therapeutisch Wirkung aufweist, als die freie Wirkstoff.

54. Verwendung der Liposomenformulierungen nach einem der Ansprüche 36-53 zur Herstellung ein Arzneimittel für ein neue ärzlich Behandlung, wobei ein therapeutisch wirkend Menge aus ein Liposomformulierung aus Vidarabin (oder ein Vidarabinderivat) eingesetzt wird.

## Revendications

1. Procédé pour la préparation des formulations en liposomes de vidarabine (ou d'un dérivé de vidarabine) appropriées pour l'administration à un mammifère, comprenant:
former une formulation en liposomes multilamellaire de vidarabine (ou d'un dérivé de vidarabine) à partir d'une solution aqueuse et un mélange de filmes lipides contenant ou pas un composant ayant une charge électrique;
lyophiliser la vidarabine (ou le dérivé de vidarabine) en liposomes;
réhydrater la vidarabine (ou le dérivé de vidarabine) en liposomes lyophilisée pour obtenir une osmolarité physiologique finale, d'abord dans une solution non-saline avec une osmolarité approximativement physiologique; et
réhydrater ensuite la vidarabine (ou le dérivé de vidarabine) en liposomes lyophilisée avec une solution saline physiologiquement acceptable.

2. Procédé selon la revendication 1, comprenant additionnellement le stade de dimensionnement de la formulation en liposomes réhydratée de vidarabine (ou d'un dérivé de vidarabine) par extrusion à travers d'une membrane poreuse.

3. Procédé selon la revendication 2, dans lequel la formulation en liposomes de vidarabine (ou d'un dérivé de vidarabine) est extrudée sous pression à travers d'une membrane de 0,8 µm.

4. Procédé selon la revendication 2, dans lequel une formulation en liposomes de vidarabine (ou d'un dérivé de vidarabine) est extrudée sous pression séquentiellement à travers de membranes de 0,8, 0,6 µm et 0,4 µm.

5. Procédé selon la revendication 1, dans lequel la formulation en liposomes de vidarabine (ou d'un dérivé de vidarabine) résultante a un diamètre d'approximativement 0,3 µm - 5,0 µm.

6. Procédé selon la revendication 3, dans lequel la formulation en liposomes de vidarabine (ou d'un dérivé de vidarabine) résultante a un diamètre d'approximativement 0,3 µm - 0,7 µm.

7. Procédé selon la revendication 4, dans lequel la formulation en liposomes de vidarabine (ou d'un dérivé de vidarabine) résultante a un diamètre d'approximativement 0,2 µm - 0,4 µm.

8. Procédé selon les revendications 3 et 4, dans lequel tout vidarabine (ou dérivé de vidarabine) non incorporé dans les liposomes est séparé avant ou après le stade d'extrusion.

9. Procédé selon la revendication 1, dans lequel la concentration du mélange des lipides dans le film lipidique est de 10 mM jusqu'à 100 mM.

10. Procédé selon la revendication 9, dans lequel la concentration du mélange des lipides dans le film lipidique est de 17 mM jusqu'à 85 mM.

11. Procédé selon la revendication 1, dans lequel un composant lipidique du mélange des filmes lipidiques est une molécule chargée.

12. Procédé selon la revendication 11, dans lequel un composant lipidique du mélange des lipides a une charge positive et est capable d'interagir avec la molécule de vidarabine (ou d'un dérivé de vidarabine).

13. Procédé selon la revendication 11, dans lequel un composant lipidique du mélange des lipides a une charge négative et est capable d'interagir avec la molécule de vidarabine (ou d'un dérivé de vidarabine).

14. Procédé selon la revendication 12, dans lequel pour l'encapsulation du monophosphate de vidarabine (ARA-AMP) la concentration de la molécule positive est d'au moins 5%.

15. Procédé selon la revendication 14, dans lequel pour l'encapsulation du monophosphate de vidarabine (ARA-AMP) la concentration de la molécule positive est approximativement 17%.

16. Procédé selon la revendication 1, dans lequel le mélange des filmes lipidiques comprend, en une proportion molaire de 0,01 à 10 : cholestérol (CHOL), phosphatidylcholine (PC), phosphatidylcholine hydrogénée (HPC), distearoylphosphatidylcholine (DSPC), sphingomyelin (SM), dioleoylphosphatidylcholine (DOPC), dioleoylphosphatidylglycerol (DOPG), phosphatidylglycerol (PG), dimirystoylphosphatidylcholine (DMPC), dipalmitoylphosphatidylcholine (DPPC), gangliosides, ceramides, phosphatidylinositol (PI), acide phosphatidique (PA), dicetylphosphate (DCP), dimirystoylphosphatidylglycerol (DMPG), stearylamine (SA), dipalmitoylphosphatidylglycerol (DPPG) et tout autre lipide synthétique.

17. Procédé selon la revendication 16, dans lequel le mélange des films lipidiques comprend un glycerophospholipide choisi entre phosphatidylcholine, dimirystoylphosphatidylcholine, dipalmitoylphosphatidylcholine; un stérol, tel que le cholestérol, présent optionnellement ; et une molécule lipidique chargée ou pas, (phosphatidylinositol, phosphatidylglycerol, stearylamine); chaque composant de la formulation de liposomes, si présent, ayant des proportions molaires relatives de 40-70%, 10-50% et 5-25%, respectivement.

18. Procédé selon la revendication 17, dans lequel, pour l'encapsulation du monophosphate de vidarabine (ARA-AMP), les composants lipidiques de phosphatidylcholine: cholestérol:stearylamine sont présents dans une proportion molaire de approximativement 10:5:3.

19. Procédé selon la revendication 1, dans lequel la vidarabine (ou le dérivé de vidarabine) lyophilisée en liposomes est réhydratée dans une solution de dextrose, galactose, mannitol, saccharose ou un autre sucre avec une concentration suffisante pour maintenir une osmolarité physiologique.

20. Procédé selon la revendication 1, dans lequel la solution saline physiologiquement acceptable du stade final est la solution 0,9% de NaCl.

21. Procédé selon la revendication 1, dans lequel la solution aqueuse utilisée pour former la formulation en liposomes multilamellaire de vidarabine (ou d'un dérivé de vidarabine) contient une concentration de vidarabine, monophosphate de vidarabine ou un autre dérivé de vidarabine de 0,06 mg/ml à 6 mg/ml.

22. Procédé selon la revendication 1, dans lequel le film lipidique inclut un dérivé de vidarabine.

23. Procédé selon la revendication 21, dans lequel le dérivé de vidarabine est le monophosphate de vidarabine (ARA-AMP).

24. Procédé selon les revendications 21, 22 et 23, **caractérisée en ce que** l'on obtient des formulations de liposomes de vidarabine (ou d'un dérivé de vidarabine) avec une efficacité d'encapsulation jusqu'à 100 %.

25. Procédé selon la revendication 24, **caractérisé en ce que** l'on obtient des formulations de liposomes de vidarabine (ou d'un dérivé de vidarabine) qui présente une stabilité dans une solution saline à 4°C supérieure à 50% et qui maintient les caractéristiques des liposomes après 45 jours d'incubation.

26. Procédé selon les revendications 1, 2 et 8, comprenant additionnellement le stade de resuspension des liposomes dans des solutions à 10% de saccharose ou 10% de tréhalose ou une autre solution isotonique de sucre après séparation du médicament extra-liposomes.

27. Procédé selon la revendication 26, comprenant additionnellement le stade de lyophilisation de la suspension de liposomes.

28. Procédé selon la revendication 27, **caractérisé en ce que** l'on obtient des formulations de liposomes qui présentent une grande stabilité dans l'état lyophilisé en termes de la morphologie des liposomes et rétention intra-liposomes du médicament.

29. Procédé selon la revendication 28, **caractérisé en ce que** l'on obtient des formulations de liposomes qui, après reconstitution avec de l'eau stérilisée et deionisée de la poudre lyophilisée, stockée sous vide à 4°C pendant 1 an, on observe des modifications non signifiantes des diamètres des liposomes et plus de 50% (pour dérivés de vidarabine solubles dans l'eau) ou d'au moins 90% (pour dérivés lipophiliques) du médicament initialement retenu dans la forme de liposomes.

30. Procédé selon la revendication 23, dans lequel les formulations de liposomes ARA-AMP, quand administrées à un patient sont non toxiques et non immunologiques.

31. Procédé selon la revendication 23, dans lequel les formulations de liposomes ARA-AMP, quand injectées de façon intraveineuse à un patient, prolongent le temps de résidence du médicament dans le courrant sanguine.

32. Procédé selon la revendication 23, dans lequel les formulations de liposomes ARA-AMP, quand injectées de façon intraveineuse à un patient, sont accumulées surtout dans le foie (dans une grande extension) et la rate.

33. Procédé selon la revendication 23, dans lequel les formulations de liposomes ARA-AMP, quand injectées de façon intraveineuse à un patient, sont accumulées en hépatocytes et cellules Kupffer.

34. Procédé selon la revendication 23, **caractérisé en ce que** l'on obtient des formulations de liposomes ARA-AMP avec un index thérapeutique supérieur en comparaison avec le médicament seul.

35. Procédé selon la revendication 23 **caractérisé en ce que** l'on obtient des formulations de liposomes ARA-AMP avec une activité thérapeutique supérieure, quand utilisées dans le traitement de l'hépatite chronique du type B ou autres maladies dans lesquelles elles sont thérapeutiquement actives, en comparaison avec le médicament seul.

36. Formulation en liposomes appropriée pour l'administration à mammifère préparée selon l'une quelconque des revendications 1-35, comprenant vidarabine, monophosphate de vidarabine ou une autre dérivé de vidarabine, **caractérisée en ce qu'**elle a une concentration de médicament de 0,06 mg/ml jusqu'à 6 mg/ml et une concentration de lipide de 17 mM jusqu'à 85 mM.

37. Formulation en liposomes de vidarabine (ou d'un dérivé de vidarabine) selon la revendication 36, dans laquelle les liposomes varient de 0,01 µm jusqu'à 5,0 µm de diamètre.

38. Formulation en liposomes de vidarabine (ou d'un dérivé de vidarabine) selon la revendication 36, dans laquelle le composant lipide comprend, dans une proportion molaire qui varient de 0,01 jusqu'à 10: cholestérol (CHOL), phosphatidylcholine (PC), phosphatidylcholine hydrogénée (HPC), distearoylphospnatidylcholine (DSPC), sphingomyelin (SM), dioleoylphosphatidylcholine (DOPC), dioleoylphosphatidylcholine (DOPG), phosphatidylglycerol (PG), dimirystoylpbosphatidylcholine (DMPC), dipalmitoylphosphatidylcholine (DPPC), gangliosides, ceramides, phosphatidylinositol (PI), acide phosphatidique (PA), dicetylphosphate (DcP), dimirystoylphosphatidylglycerol (DMPG), stearylamine (SA), dipalmitoylphosphatidylglycerol (DPPG) et tout autre lipide synthétique.

39. Formulation en liposomes de vidarabine (ou d'un dérivé de vidarabine) selon la revendication 36, dans laquelle le composant lipide comprend un glycerophospholipide choisi parmi phosphatidylcholine, dimiristoylphosphatidylcholine, dipalmitoylphosphatidylcholine; un stérol, tel que le cholestérol, optionnellement présent; et une molécule lipidique chargée ou pas (phosphatidylinositol, phosphatidylglycerol, stearylamine); chaque composant de la formulation en liposomes, si présent, étant dans les proportions molaires de 40-70%, 10-50% et 5-25%, respectivement.

40. Formulation en liposomes selon la revendication 39, dans laquelle, pour l'encapsulation de monophosphate de vidarabine (ARA-AMP), le composant lipide comprend phosphatidylcholine:cholestérol:stearylamine dans des proportions molaire relatives de 40-70%, 10-50% et 5-25%, respectivement.

41. Formulation en liposomes selon la revendication 40, dans laquelle, pour l'encapsulation de monophosphate de vidarabine (ARA-AMP), les composants lipides de phosphatidylcholine; cholestérol:stearylamine sont présents dans une proportion molaire de approximativement 10:5:3.

42. Formulation en liposomes selon la revendication 40, dans laquelle la concentration de la molécule positive (dans des termes de proportions molaires relatives de lipides) est d'au moins 15%.

43. Formulation en liposomes de vidarabine (ou d'un dérivé de vidarabine) selon la revendication 36, ayant approximativement une osmolarité physiologique.

44. Formulation en liposomes de vidarabine selon la revendication 36, dans laquelle le dérivé de vidarabine est le monophosphate de vidarabine (ARA-AMP).

45. Formulation en liposomes de vidarabine (ou d'un dérivé de vidarabine) selon les revendications 36-44, qui est non toxique et non immunologique.

46. Formulation en liposomes de vidarabine (ou d'un dérivé de vidarabine) selon les revendications 36-44, **caractérisée en ce que** le temps de résidence dans le courant sanguine est augmenté, en comparaison avec le médicament seul.

47. Formulation en liposomes de vidarabine (ou d'un dérivé de vidarabine) selon les revendications 36-44, **caractérisée en ce que** l'accumulation se vérifie surtout dans le foie (dans une grande extension) et dans la rate.

48. Formulation en liposomes de vidarabine (ou d'un dérivé de vidarabine) selon les revendications 36-44, **caractérisée en ce que** l'accumulation du médicament dans le foie et dans la rate est élevée en comparaison avec le médicament seul.

49. Formulation en liposomes de vidarabine (ou d'un dérivé de vidarabine) selon les revendications 36-44, **caractérisée en ce que** les niveaux du médicament dans la moelle des os sont inférieurs en comparaison avec le médicament seul.

50. Formulation en liposomes de vidarabine (ou d'un dérivé de vidarabine) selon les revendications 36-44, **caractérisée en ce que** l'accumulation se vérifie dans les hépatocytes et cellules Kupffer.

51. Formulation en liposomes de vidarabine (ou d'un dérivé de vidarabine) selon les revendications 36-44, **caractérisée en ce que** l'accumulation du médicament dans les cellules du foie est élevée en comparaison avec le médicament seul.

52. Formulation en liposomes de vidarabine (ou d'un dérivé de vidarabine) selon les revendications 36-44, **caractérisée en ce que** l'index thérapeutique est élevé en comparaison avec le médicament seul.

53. Formulation en liposomes de vidarabine (ou d'un dérivé de vidarabine) selon les revendications 36-44, **caractérisée en ce que** l'activité thérapeutique est élevée, si utilisée dans le traitement de l'hépatite chronique du type B ou dans d'autres maladies dans lesquelles elles sont thérapeutiquement actives, en comparaison avec le médicament seul.

54. Utilisation des formulations de liposomes selon l'une quelconque des revendications 36-53, pour la manufacture d'un médicament pour un nouveau traitement médical, dans laquelle on utilise une quantité thérapeutiquement effective d'une formulation en liposomes de vidarabine (ou d'un dérivé de vidarabine).
